# EUROPEAN PATENT APPLICATION

(11) **EP 3 744 824 A1**
(43) Date of publication of application: **02.12.2020**
(21) Application number: 19743730.4
(22) Date of filing: 15.01.2019
(51) Int. Cl.: C12M 1/00, C12M 1/36, C12N 1/00, C12N 5/071

(54) **CARRIER FOR CELL CULTURE AND CELL CULTURE VESSEL**

(30) Priority: 24.01.2018 JP 2018009460
(71) Applicant: Yokogawa Electric Corporation, Tokyo 180-8750 (JP)
(72) Inventor: MOGI Takeyuki, Musashino-shi, Tokyo 180-8750 (JP)
(74) Representative: Grünecker Patent- und Rechtsanwälte PartG mbB
(86) International application number: PCT/JP2019/000880
(87) International publication number: WO 2019/146444

(57) **Abstract**

A cell culture carrier includes a stimulus-responsive substrate for cell culture comprising a stimulus-responsive polymer and an external signal receiving material, an active substance is supported on the stimulus-responsive substrate for cell culture.

## Description

### [Technical Field]

The present invention relates to a cell culture carrier and a cell culture container.

### [Background Art]

In recent years, with progress in regenerative medicine technology and cancer treatment technology, preparing large numbers of cells for clinical use has been required. In order to culture cells, it may be necessary to periodically add components which easily decompose to the medium(a cell culture solution) or change the required components which differ in accordance with a state of the cells. Medium exchange involves the risk of contamination of a culture system. For this reason, it is desirable that a process be able to be performed automatically without human intervention as far as possible.

For example, Non-Patent Literature 1 describes a method in which a carrier having an active substance such as a cytokine supported therein is placed around cells in a culture container and the active substance is supplied through diffusion from the carrier into the medium.

Also, Patent Literature 1 describes a method in which a small amount of cells is placed at a desired position in a minute flow path device and an effective drug sensitivity test for an anticancer agent or the like is performed. In the method described in Patent Literature 1, a microfluidic device is used and an active substance such as a cytokine is supplied to the medium in a device using a pump.

### [Citation List]

### [Patent Literature]

[Patent Literature 1]
Japanese Patent No. 5881031

### [Non-Patent Literature]

[Non-Patent Literature 1]
Lotz, S et al. Sustained Levels of FGF2 Maintain Undifferentiated Stem Cell Cultures with Biweekly Feeding. PLoS One. 8(2): e56289 (2013).

### [Summary of Invention]

### [Technical Problem]

The method described in Non-Patent Literature 1 can be performed through a simple operation simply by placing a carrier in the medium. However, since an active substance is continuously released through diffusion, there is a problem that it is difficult to control the supply of the active substance.

The method described in Patent Literature 1 allows control of the supply of the active substance in detail by controlling the driving of a pump outside a microfluidic device. However, a microfluidic device having such a mechanism has a problem that a structure is complicated and a manufacturing cost thererof is high. Furthermore, since a mechanism such as a pump occupies a large area, there is a problem that a large culture space cannot be secured and a large amount of cells cannot be cultured.

The present invention was made in view of the above circumstances, and an object of the present invention is to provide a cell culture carrier and a cell culture container in which detailed control of supply of an active substance can be performed, in which a large amount of cells can be cultivated, and which can be produced at low cost.

### [Solution to Problem]

That is to say, the present invention includes the invention of the following [1] to [14].
[1] A cell culture carrier including: a stimulus-responsive substrate for cell culture comprising a stimulus-responsive polymer and an external signal receiving material, an active substance is supported on the stimulus-responsive substrate for cell culture.
[2] The cell culture carrier according to [1], wherein the active substance is contained in a porous substrate, and the porous substrate and the active substance are supported in the stimulus-responsive substrate for cell culture.
[3] The cell culture carrier according to [1] or [2], wherein the stimulus-responsive polymer is a temperature responsive polymer.
[4] The cell culture carrier according to any one of [1] to [3], wherein the external signal receiving material is an organic compound, a metal structure, or a carbon material.
[5] The cell culture carrier according to [4], wherein the metal structure is gold nanorods.
[6] The cell culture carrier according to any one of [1] to [5], wherein the active substance is a protein such as an enzyme, a cytokine, a differentiation inducing factor, and an antibody, a peptide such as a hormone, a small-molecular compound such as an antibiotic, an amino acid, glucose, and retinoic acid, a nucleic acid such as DNA, nanoparticles, or liposomes.
[7] The cell culture carrier according to any one of [1] to [6], wherein an external signal received by the external signal receiving material is near-infrared light.
[8] The cell culture carrier according to [2], wherein a part of the porous substrate containing the active substance is covered with the stimulus-responsive substrate for cell culture and the other parts are covered with an active substance impermeable layer.
[9] The cell culture carrier according to [1], wherein the external signal receiving material is blended with the stimulus-responsive substrate for cell culture so that a concentration distribution is formed.
[10] The cell culture carrier according to [1], further includes: an injection port through which the active substance is injected; and a sealing section formed of a material impermeable to an active substance and configured to seal the injection port are provided.
[11] A cell culture container includes: a cell culture carrier comprising a stimulus-responsive substrate for cell culture comprising a stimulus-responsive polymer and an external signal receiving material, an active substance is supported on the stimulus-responsive substrate for cell culture; and a cell culture section.
[12] The cell culture container according to [11], wherein the active substance is contained in a porous substrate and the porous substrate and the active substance are supported in the stimulus-responsive substrate for cell culture.
[13] The cell culture container according to [11] or [12], wherein the stimulus-responsive polymer is a temperature responsive polymer.
[14] The cell culture container according to any one of [11] to [13], wherein the external signal receiving material is an organic compound, a metal structure, or a carbon material.
[15] The cell culture container according to [14], wherein the metal structure is gold nanorods.
[16] The cell culture container according to any one of [11] to [15], wherein the active substance is a protein such as an enzyme, a cytokine, a differentiation inducing factor, and an antibody, a peptide such as a hormone, a small-molecular compound such as an antibiotic, an amino acid, glucose, and retinoic acid, a nucleic acid such as DNA, nanoparticles, or liposomes.
[17] The cell culture container according to any one of [11] to [16], wherein an external signal received by the external signal receiving material is near-infrared light.
[18] The cell culture container according to [12], wherein a part of the porous substrate containing the active substance is covered with the stimulus-responsive substrate for cell culture and the other parts are covered with an active substance impermeable layer.
[19] The cell culture container according to [11], wherein the external signal receiving material is blended with the stimulus-responsive substrate for cell culture so that a concentration distribution is formed.
[20] The cell culture container according to [11], further comprising: an injection port through which the active substance is injected; and a sealing section formed of a material impermeable to an active substance and configured to seal the injection port are provided.

Further features and aspects of the present invention will be apparent from the detailed description of the embodiments set forth below with reference to the accompanying drawings.

### [Advantageous Effects of Invention]

According to the present invention, it is possible to provide a cell culture carrier and a cell culture container in which detailed supply control of an active substance can be performed, in which a large amount of cells can be cultivated, and which can be produced at low cost.

### [Brief Description of Drawings]

FIG. 1 is a schematic diagram illustrating an example of a cell culture carrier of the present invention.
FIG. 2 is a schematic diagram illustrating an example of the cell culture carrier of the present invention.
FIG. 3 is a schematic diagram illustrating an example of the cell culture carrier of the present invention.
FIG. 4 is a schematic diagram illustrating an example of the cell culture carrier of the present invention.
FIG. 5 is a schematic diagram illustrating an example of the cell culture carrier of the present invention.
FIG. 6 is a schematic diagram illustrating an example of the cell culture carrier of the present invention
FIG. 7 is a schematic diagram for explaining a cell culture method using an example of the cell culture container of the present invention.
FIG. 8 is a schematic diagram illustrating an example of the cell culture container of the present invention.
FIG. 9 is a schematic diagram illustrating an example of the cell culture container of the present invention.
FIG. 10 is a schematic diagram illustrating an example of the cell culture container of the present invention.
FIG. 11 is a schematic diagram illustrating an example of the cell culture container of the present invention.
FIG. 12 is a schematic diagram illustrating an example of the cell culture container of the present invention.
FIG. 13 is a schematic diagram illustrating an example of the cell culture container of the present invention.
FIG. 14 is a schematic diagram illustrating an example of the cell culture container of the present invention.
FIG. 15 is a schematic diagram illustrating an example of the cell culture container of the present invention.

### [Description of Embodiments]

### <Cell culture carrier>

An embodiment includes a cell culture carrier in which an active substance is supported in a stimulus-responsive substrate for cell culture having a stimulus-responsive polymer and an external signal receiving material. A theory assumed as the reason why the diffusion of the active substance can be switched between an aggregated state and a swollen state of the stimulus-responsive polymer will be described below. Here, the present invention is not limited to this theory.

In the cell culture carrier in the embodiment, the active substance is supported in the stimulus-responsive substrate for cell culture. In the cell culture carrier, the stimulus-responsive polymer in the stimulus-responsive substrate for cell culture is aggregated before an external signal is received. In such an aggregated state, the solubility of the polymer in water is low because a density of the polymer is high and the hydrophobicity of the polymer is high. Furthermore, since the active substance supported in the cell culture carrier does not diffuse outside of the carrier, the active substance remains supported in the cell culture carrier. The active substance is stored in the cell culture carrier.

If an external signal is received, the aggregation of the stimulus-responsive polymer in the stimulus-responsive substrate for cell culture is broken down. For this reason, the solubility of the polymer in water is improved, the polymer swells, and the active substance supported in the cell culture carrier diffuses outside of the cell culture carrier. Furthermore, if an input of the external signal is stopped, the stimulus-responsive substrate for cell culture is brought into an aggregated state again and the diffusion of the active substance is stopped. It is possible to reversibly control the aggregation of the stimulus-responsive polymer by controlling the input of the external signal. That is to say, in the cell culture carrier in the embodiment, it is possible to control the diffusion of the active substance in detail by controlling the input of the external signal.

The cell culture carrier in the embodiment will be described in detail below.

### <<First embodiment>>

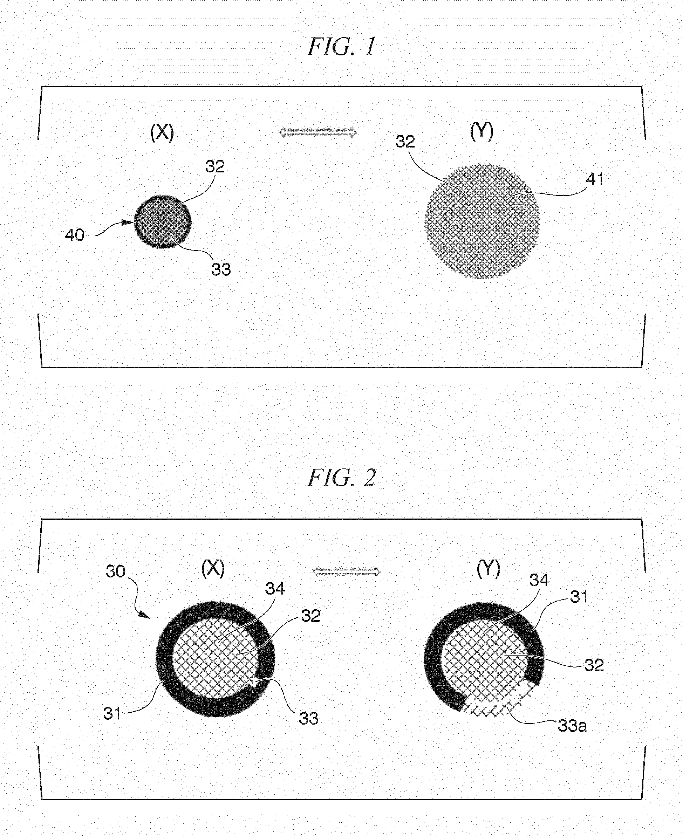

FIG. 1 is a schematic diagram illustrating a cross section of a cell culture carrier 40 in a first embodiment. The cell culture carrier 40 illustrated in FIG. 1 is in a state in which an active substance 32 is supported in a stimulus-responsive substrate for cell culture 33. A structure is simple and efficient production is possible by directly supporting the active substance 32 on the stimulus-responsive substrate for cell culture 33. If an external signal is input to the cell culture carrier 40, the cell culture carrier 40 is brought into a swollen state 41 in which the cell culture carrier 40 is swollen as a whole.

A symbol (X) in FIG. 1 represents a state in which the stimulus-responsive polymer is aggregated and the diffusion of the active substance is stopped. Furthermore, a symbol (Y) in FIG. 1 represents a state in which the stimulus-responsive polymer is swollen and the active substance can diffuse. In the embodiment, it is possible to reversibly control a diffusion state (Y) and stopping of diffusion (X) of the active substance by controlling the input of an external signal. Also in the following drawings, it is assumed that (X) and (Y) mean the same states.

As an example, in the case of the aggregated state represented by the symbol (X) before the input of an external signal, if the external signal is received and the stimulus-responsive polymer in the stimulus-responsive substrate for cell culture 33 is in a non-aggregated state 41 (or a swollen state), the solubility of the polymer in water is improved. Thus, the supported active substance 32 diffuses outside of the carrier.

As another example, in the case of the non-aggregated state represented by the symbol (Y) before the input of the external signal, if the external signal is received and the stimulus-responsive polymer in the responsive substrate for cell culture 33 is in an aggregated state, the solubility of the polymer in water is reduced. Thus, the supported active substance 32 remains supported in the carrier.

### • Stimulus-responsive polymer

An external stimulus changes a structure of the stimulus-responsive polymer and changes the permeability of the active substance. The stimulus-responsive polymer may be a linear polymer or a branched polymer. The stimulus-responsive polymer molecules used may be cross-linked. A polymer brush structure using a stimulus-responsive polymer may be formed and used. When the active substance has a small molecular size, it is desirable that the stimulus-responsive polymer have a high density due to cross-linking, a polymer brush structure, or the like in view of sealing efficiency.

As the stimulus-responsive polymer, polymers capable of responding to various stimuli such as temperature, light, pH, a magnetic field, an electric field, ultrasonic waves, redox conditions, and a molecular concentration can be appropriately used. In the embodiment, even if the temperature responsive polymer is locally heated, since the temperature thereof converges to a culture temperature by thermal equilibrium and also the temperature responsive polymer has little effect on the culture environment, the temperature responsive polymer has no adverse effect on cells. Thus, it is desirable to use temperature responsive polymers.

As the temperature responsive polymers, lower critical solution temperature (LCST) type polymers and upper critical solution temperature (UCST) type polymers can be used. As the lower critical solution temperature (LCST) type polymers, polyamide type LCST type polymers, polyether type LCST type polymers, phosphoester type LCST type polymers, polymers having a protein incorporated therein, and the like can be used. To be more specific, as the lower critical solution temperature (LCST) type polymers, poly(N-isopropylacrylamides), poloxamers, poly(N-vinylcaprolactams), polymethylvinylethers, methylcelluoses, elastin-containing polymers, and the like can be used. Furthermore, as the upper critical solution temperature (UCST) type polymers, poly(allylamine-co-allyl ureas), poly(acrylamide-co-acrylonitriles), hydroxypropyl celluloses, poloxamers such as poloxamer 407, methacrylamide polymers, polymethacrylates having a sulfobetaine group, and the like can be used. These polymers can be used independently or a combination of a plurality of these polymers can be used. In addition, the LCST type polymers or the UCST type polymers may be block copolymers formed using two or more monomers selected from the group consisting of isopropyl acrylamide, caprolactam, allyl amine, allyl urea, sulfobetaine, ethylene glycol, methacrylates, styrene, norbornene, phosphazene, methyl vinyl ether, acrylonitrile, and lactides. Phase transition temperatures of these block copolymers can be adjusted in accordance with a type and ratio of amounts of monomers to be used. Furthermore, a combination of LCST type polymers with UCST type polymers can also be used for the temperature responsive polymer. The phase transition temperatures of these temperature responsive materials can be appropriately adjusted in accordance with an amount of functional groups to be introduced or the like. For example, as the functional groups to be introduced, alkyl groups, amide groups, piperazine groups, pyrrolidine groups, acetal groups, ketal groups, oxazoline groups, oxyethylene groups, sulfonate groups, alcohol groups, sulfobetaine groups, uracil groups, ureido groups, and glycinamide groups can be used. Stimulus response temperatures are preferably within the range of 20 °C to 60 °C. It is desirable that at least one of the stimulus response temperatures of the temperature responsive polymer be a culture temperature or higher. Particularly, in a culture of animal cells of humans or the like, a culture temperature is 37 °C. Thus, the stimulus response temperature is preferably 38 °C or higher and 50 °C or lower. It is possible to reversibly control the states (X) and (Y) before and after the input of the external signal by selectively using an LCST type and a UCST type.

### • External signal

The external signal is preferably light or a magnetic field from the viewpoint that in this case no wiring is required. As the light, near-infrared light which does not easily affect cells is preferable. Above all, it is desirable that a wavelength of light used as an external signal be a wavelength which is difficult for water molecules to absorb. For example, the wavelength of the light used as an external signal may be a wavelength of 650 nm or more and 950 nm or less; 1000 nm or more and 1350 nm or less; and 1500 nm or more and 1800 nm or less.

### • External signal receiving material

The external signal receiving material is appropriately determined through a combination of an external signal and a stimulus-responsive polymer. For example, when near-infrared light is used for the external signal and a temperature-responsive polymer is used for the stimulus-responsive polymer, as the external signal receiving material, organic materials and inorganic materials in which the near-infrared light is efficiently converted into heat are used. As such materials, organic compounds, metal structures, or carbon structures may be used. It is desirable that the metal structures and the carbon structures be fine. By making the metal structures and the carbon structures fine, these structures can be easily made uniform without sedimentation and a surface area can be increased. Thus, it is possible to improve the absorption efficiency of the near-infrared light.

Since a signal is received and the surroundings of the external signal receiving material is locally heated using such materials as the external signal receiving material, the structure of the temperature responsive polymer is changed and it is possible reversibly control the diffusion and stopping of diffusion of the active substance.

### •• Organic compound

In the embodiment, it is desirable that the organic compound be an organic dye. As the organic dye, cyanine dyes, phthalocyanine dyes, naphthalocyanine compounds, nickel dithiolene complexes, squarylium dyes, quinone compounds, diimmonium compounds, azo compounds, porphyrin compounds, dithiol metal complexes, naphthoquinone compounds, diimmonium compounds, and the like may be used. Furthermore, as the organic compound, resin particles containing an organic dye may be used. A shape of the organic compound is not limited to particles in some cases. By kneading the organic dye with a resin, the leakage of the organic dye into a culture solution can be minimized, which is effective in preventing an influence of the organic dye on a culture object such as cells.

### •• Metal structure

In the embodiment, as the metal structure, metal nanorods are preferable and gold nanorods are particularly preferable because in this case an absorption wavelength of light can be controlled using an aspect ratio.

### • Carbon material

A carbon structure is preferable as a carbon material. Carbon nanotubes, fullerenes, carbon nanowires, and the like can be used as the carbon structure.

When a magnetic field is utilized for an external signal, it is desirable that a magnetic material such as magnetic nanoparticles be used for an external signal receiving substance . Magnetic nanoparticles generate heat due to a load of an alternating current (AC) magnetic field.

The bonding of the stimulus-responsive polymer and the external signal receiving material, cross-linking between molecules of the stimulus-responsive polymer, and fixing of the stimulus-responsive substrate for cell culture to the active substance can be performed using various functional groups and cross-linking agents reacting with the functional groups.

In the embodiment, the cross-linking agent may be a homo-bifunctional cross-linking agent, a hetero-bifunctional cross-linking agent, or a multifunctional cross-linking agent having three or more functional groups.

If the functional group is a primary amine, a cross-linking agent containing an NHS ester, carbodiimide, aldehyde, isothiocyanate, isocyanate, acyl azide, sulfonyl chloride, glyoxal, epoxide, oxirane, carbonate, aryl halide, imide ester, anhydride, fluoroester, or the like can be used.

If the functional group is a carboxyl group, a carbodiimide or the like can be used.

If the functional group is a sulfhydryl group, a cross-linking agent containing a maleimide, haloacetic acid, pyridyl disulfide, thiosulfone, vinyl sulfone, or the like can be used.

If the functional group is an aldehyde group, a cross-linking agent containing hydrazide, an alkoxyamine, or the like can be used.

Also, a photoreactive group such as a diazirine or arylazide may be used. Chemoselective ligation such as with azide-alkyne or azide-phosphine may be used. A molecule such as polyethylene glycol or DNA may be used as a spacer.

Furthermore, various members may be activated through application of energy such as in electron beam irradiation and used for bonding or cross-linking.

### • Active substance

In the embodiment, as an active substance 22, an enzyme, a cytokine, a differentiation inducing factor, a protein such as an antibody, a peptide such as a hormone, an antibiotic, a small molecular compound such as an amino acid, glucose, or retinoic acid, a nucleic acid such as DNA, a nanoparticle, a liposome, or the like can be used without particular limitation. Furthermore, the active substance 22 may be a biomaterial extract or a cell secretion product. The active substance 22 is not limited to a chemical substance and may be a virus. The cell secretion product may be supplied by embedding cells for secreting an active substance in a porous substrate and coculturing with cells to be cultured. Thus, it is also possible to supply unknown active substance to the cells to be cultured.

### <<Second embodiment>>

FIG. 2 is a schematic diagram illustrating a cross section of a cell culture carrier 30 in a second embodiment. The cell culture carrier 30 illustrated in FIG. 2 includes a stimulus-responsive substrate for cell culture 33, a porous substrate 34, an active substance 32, and an active substance impermeable layer 31. The active substance 32 is contained in the porous substrate 34. In the embodiment, a part of the porous substrate 34 containing the active substance 32 is covered with the stimulus-responsive substrate for cell culture 33 and the other part thereof is covered with the active substance impermeable layer 31. It is desirable that the active substance impermeable layer 31 be an organic material such as a hydrophobic polymer and it is desirable that the active substance 32 be not released from a portion other than the portion covered with the stimulus-responsive substrate for cell culture 33. The active substance impermeable layer 31 may be any layer as long as an active substance is impermeable and non-adsorptive with respect to the layer, an inorganic material such as a metal thin film may be used as a material other than a hydrophobic polymer, and a porous substrate may be cross-linked using a cross-linking agent. Hydrophilic coating may be applied if necessary.

By adjusting a location at which the stimulus-responsive substrate for cell culture 33 is placed and a covered area, it is possible to perform control such that an amount of diffusion and a diffusion direction of the active substance 32 give a desired amount of supply.

As illustrated in (Y) of FIG. 2, if an external signal is input to the cell culture carrier 30, only the place in which the stimulus-responsive substrate for cell culture 33 is arranged is in the swelled state 33a. Since the active substance 32 is released through diffusion only from the portion in the swelled state 33a, it is possible to control the amount of diffusion and the diffusion direction of the active substance 32 to a desired amount of supply.

### • Porous substrate

In the embodiment, the active substance 32 is contained in a base substrate. It is desirable that the base substrate be a porous substrate. The porous substrate may be a chemical gel based on covalent bonds and a physical gel based on non-covalent intermolecular forces or the like. These chemical and physical gels may be gels contributing to the formation of a gel structure. To be specific, as the porous substrate, a hydrogel formed of sugar chains such as agarose, dextrin, pectin, sodium alginate, or xanthan gum, a hydrogel formed of a protein such as collagen, hyaluronic acid, elastin, or gelatin, a hydrogel formed of a synthetic polymer such as a polyacrylamide or polyethylene glycol, or a silicone hydrogel; or an inorganic material such as mesoporous carbon, mesoporous aluminosilicate, or mesoporous silica can be used. These composite materials may be used for the porous substrate.

In the embodiment, it is desirable that the active substance 32 be contained in the porous substrate 34.

### <<Third embodiment>>

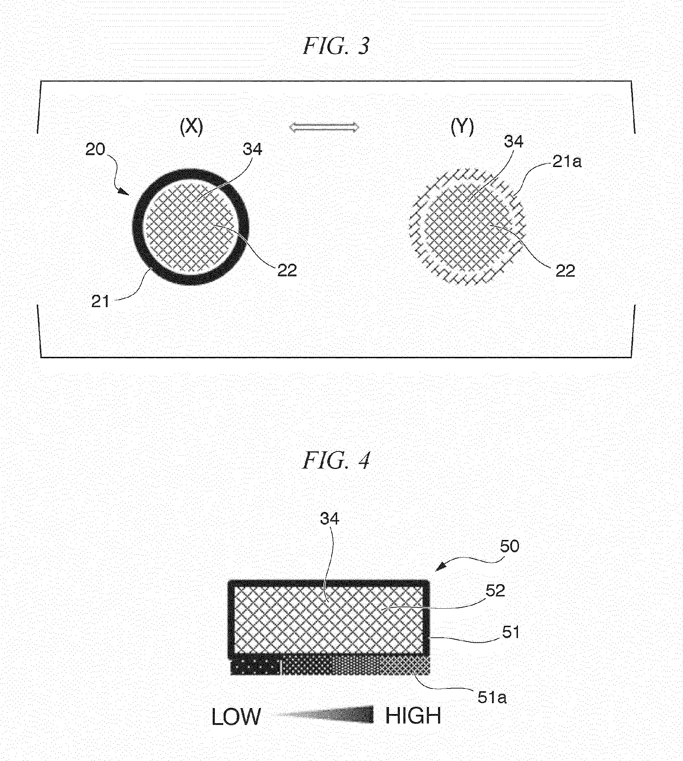

FIG. 3 is a schematic diagram illustrating a cross section of a cell culture carrier 20 in a third embodiment. The cell culture carrier 20 illustrated in FIG. 3 includes a stimulus-responsive substrate for cell culture 21, a porous substrate 34, and an active substance 22. The active substance 22 is contained in the porous substrate 34. In the cell culture carrier 20 in the embodiment, the stimulus-responsive substrate for cell culture 21 is covered with the active substance 22 and the active substance 22 is stored in the stimulus-responsive substrate for cell culture 21.

The symbol (X) in FIG. 3 represents a state in which a stimulus-responsive polymer is aggregated and the diffusion of an active substance is stopped. Furthermore, the symbol (Y) in FIG. 3 represents a state in which a stimulus-responsive polymer is swelled and an active substance can diffuse. In the embodiment, it is possible to reversibly control a diffusion state (Y) and a diffusion stop (X) of the active substance by controlling an input of an external signal.

As an example, in the case of an aggregated state illustrated in the symbol (X) before the input of the external signal, if the external signal is received and a stimulus-responsive polymer in the stimulus-responsive substrate for cell culture 21 is in a non-aggregated state 21a, the solubility of the polymer in water is improved. Thus, the supported active substance 22 diffuses outside of the carrier.

As another example, in the case of a non-aggregated state illustrated in the symbol (Y) before the input of the external signal, if the external signal is received and a stimulus-responsive polymer in the cell culture responsive substrate 21 is in an aggregated state, the solubility of the polymer in water is reduced. The supported active substance 22 remains held in the carrier.

### <<Fourth embodiment>>

FIG. 4 is a schematic diagram illustrating a cross section of a cell culture carrier 50 in a fourth embodiment. The cell culture carrier 50 illustrated in FIG. 4 is in a state in which an external signal receiving material 52 is blended with a stimulus-responsive substrate for cell culture 51 so that a concentration distribution is formed and a porous substrate 34 containing an active substance is covered with the mixture. A symbol 51a indicates a gradient of the concentration distribution. Due to the concentration distribution being present, diffusion rates of active substances can be differentiated even if the same external signal is input. For this reason, it is possible to spatially control the concentration distribution of the active substance in a cell culture container.

### <<Fifth embodiment>>

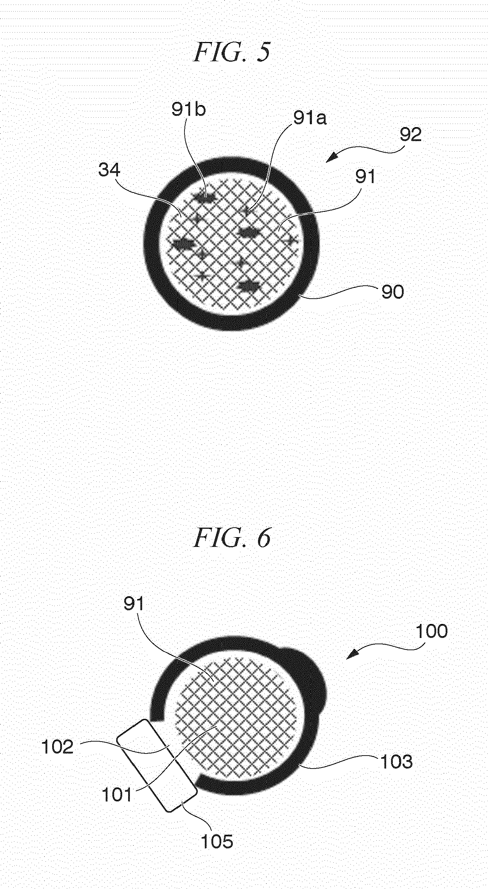

FIG. 5 is a schematic diagram illustrating a cross section of a cell culture carrier 92 in a fifth embodiment. In the cell culture carrier 92 illustrated in FIG. 5, an active substance 91 is covered with a stimulus-responsive substrate for cell culture 90. The active substance 91 is contained in a porous substrate 34. A plurality of types of active substances 91a and active substances 91b are stored in the active substance 91 in a mixed state.

### <<Sixth embodiment>>

FIG. 6 is a schematic diagram illustrating a cross section of a cell culture carrier 100 in a sixth embodiment. In the cell culture carrier 100 illustrated in FIG. 6, a porous substrate 101 containing an active substance is covered with a stimulus-responsive substrate for cell culture 103. The cell culture carrier 100 has a surface having an injection port 102 formed therein and a desired active substance 91 can be injected thereto. If the injection port 102 is sealed with a sealing section 105 formed of a material impermeable to an active substance such as a hydrophobic polymer after the active substance is injected, the effect of reducing the leakage of the active substance 91 can be obtained.

### <Cell culture container>

The cell culture container in the embodiment includes the cell culture carrier in the embodiment and a cell culture section.

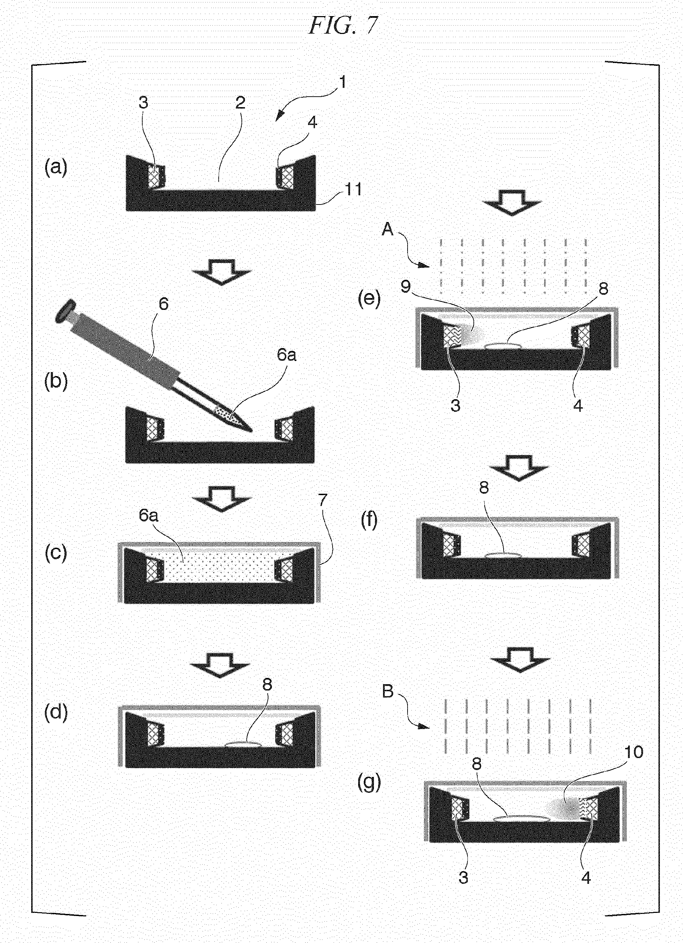

FIG. 7 is a schematic diagram illustrating a cross-sectional view of an example of a cell culture container 1 in the embodiment. The cell culture container 1 includes a cell culture section 11 and cell culture carriers 3 and 4. The cell culture section 11 includes a cell accommodation section 2.

Known materials such as existing culture dishes, wells of culture plates, and Lab-on-a-chips can be used for the cell accommodation section 2. A surface of the cell accommodation section 2 may be coated to prevent cell adhesion or desorption, nonspecific protein adsorption, and the like.

While describing the cell culture container 1 in the embodiment, a cell culture method in which the cell culture container 1 in the embodiment is used will be described.

In the cell culture container 1 illustrated in (a) of FIG. 7, the cell culture carrier 3 supports a cell inducing factor 9 as an active substance and the cell culture carrier 4 supports a growth factor 10 as an active substance.

Subsequently, as illustrated in (b) of FIG. 7, a suspension solution 6a is introduced into the cell accommodation section 2 using a pipette 6. Subsequently, as illustrated in (c) of FIG. 7, the cell culture section 11 is covered with a lid 7 and cells are cultured. Subsequently, as illustrated in (d) of FIG. 7, cells are adhered to the cell accommodation section 2 and a cell population 8 is formed.

The cell culture carrier 3 contains gold nanorods having an absorption peak at a wavelength near 780 nm as an external signal receiving substance. The cell culture carrier 4 contains gold nanorods having an absorption peak at a wavelength near 900 nm as an external signal receiving substance.

As illustrated in (e) of FIG. 7, if near-infrared light A of 780 nm is radiated as an external signal, the cell inducing factor 9 diffuses from the cell culture carrier 3. Furthermore, if the irradiation of the near-infrared light A is stopped, the diffusion of the cell inducing factor 9 is stopped.

As illustrated in (f) of FIG. 7, the cell population 8 moves in a direction of the cell culture carrier 3 through the diffusion of the cell inducing factor 9.

As illustrated in (g) of FIG. 7, if near-infrared light B of 900 nm is radiated as an external signal, the growth factor 10 diffuses from the cell culture carrier 4. Furthermore, if the irradiation of the near-infrared light B is stopped, the diffusion of the growth factor 10 is stopped.

Cells proliferates through the diffusion of the growth factor 10.

### • Cells

Cells to be cultured may be any of stem cells such as mesenchymal stem cells, ES cells and iPS cells, progenitor cells, differentiated cells such as liver, established cells such as tumor-derived cells, and cells other than mammalian cells. Bacteria, yeast, or fungi may be used.

### <<Other embodiments of cell culture container>>

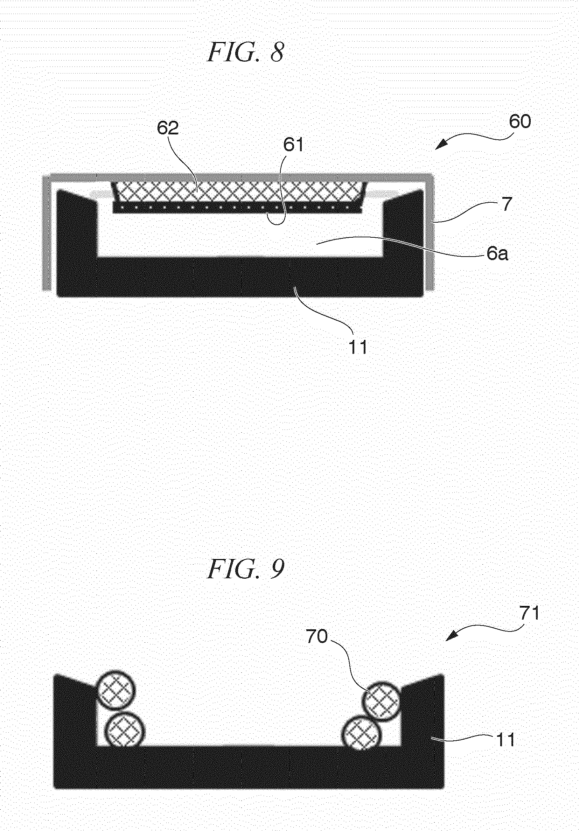

FIG. 8 is a diagram illustrating an embodiment of the cell culture container. A cell culture container 60 includes a stimulus-responsive substrate for cell culture 61 and a porous substrate 62 containing an active substance inside a container of a lid 7. The porous substrate 62 is arranged to come into contact with the lid 7. The stimulus-responsive substrate for cell culture 61 is arranged to come into contact with a cell suspension solution 6a in the cell culture section 11.

The active substance contained in the porous substrate 62 may have a concentration gradient in the porous substrate 62. Since an external signal is input from the lid 7 side of the cell culture container 60, it is possible to supply the active substance.

FIG. 9 is a diagram illustrating an embodiment of the cell culture container. A plurality of bead-shaped cell culture carriers 70 are installed in a cell culture section 11 of a cell culture container 71. Since different active substances are supported in the cell culture carriers 70, it is possible to supply various types of active substances.

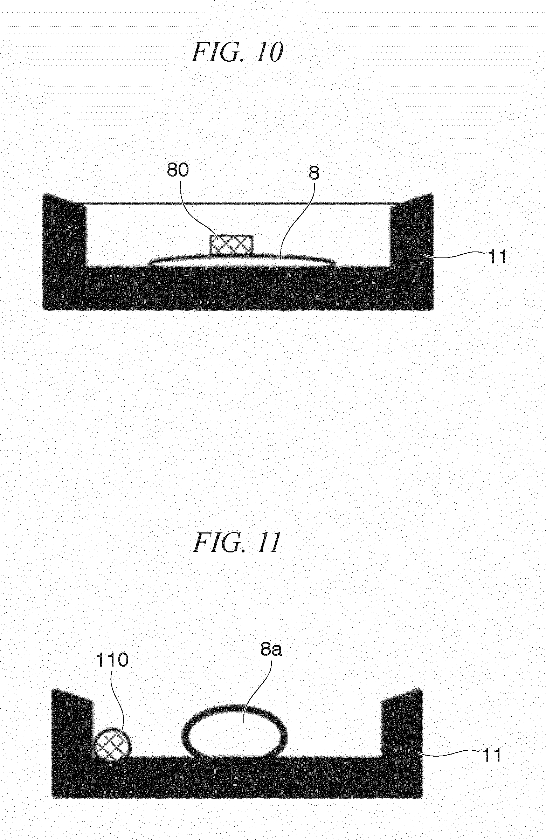

FIG. 10 is a diagram illustrating an embodiment of the cell culture container. When a cell culture carrier 80 is installed in a cell culture section 11, the cell culture carrier 80 may placed above a cell population 8.

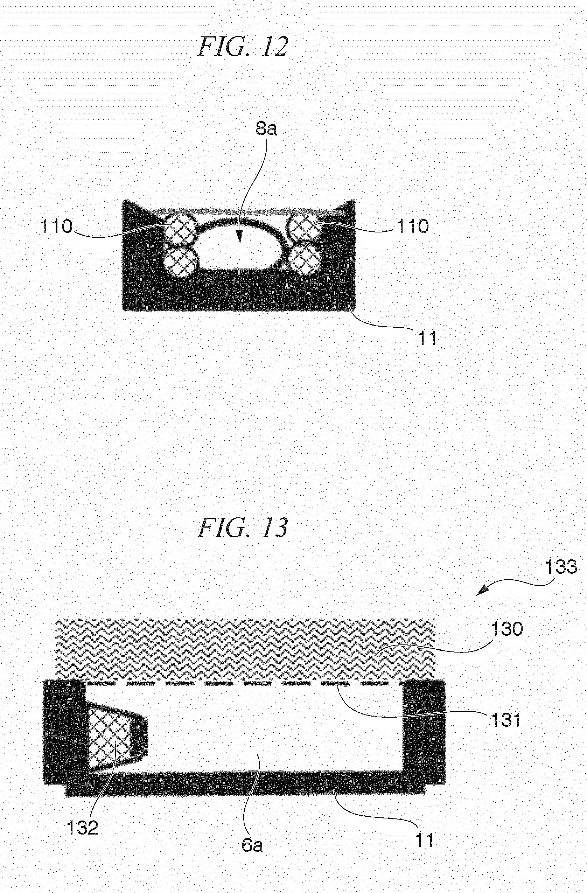

FIG. 11 is a diagram illustrating an embodiment of the cell culture container. A cell population may be a three-dimensional cell population 8a present in a cell culture section 11. A cell culture carrier 110 may not be in contact with the three-dimensional cell population 8a. As illustrated in FIG. 12, a plurality of cell culture carriers 110 may be in contact with a three-dimensional cell population 8a.

FIG. 13 is a diagram illustrating an embodiment of the cell culture container. A cell culture container 133 includes a cell culture section 11, a cell culture carrier 132, a semi-permeable film 131, a medium 130, and a cell suspension solution 6a. The medium 130 refluxes above the semi-permeable film 131. According to the embodiment, small molecules such as nutrients and waste products can be exchanged through the semi-permeable film. Thus, the environment in the cell culture section 11 can be maintained in an appropriate state in which cells are kept alive. Furthermore, since the medium 130 refluxes above the semi-permeable film 131, it is not necessary to replace the medium 130. A polymer material which cannot pass through the semi-permeable film 131 can be supplied by supporting the polymer material in a cell culture carrier 132.

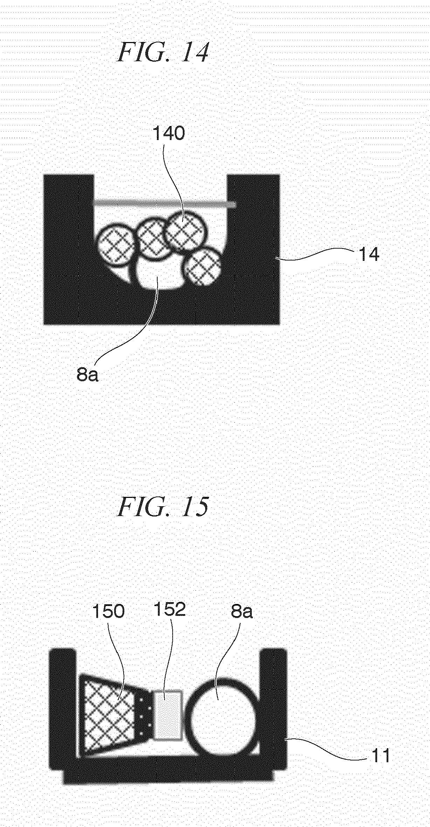

FIG. 14 is a diagram illustrating an embodiment of the cell culture container. A cell accommodation section 14 has a bottom having a concave shape. It is possible to form a three-dimensional cell population 8a using the cell accommodation section 14 of the concave shape and it is possible to continuously perform the stimulation from the cell culture carrier 140.

FIG. 15 is a diagram illustrating an embodiment of the cell culture container. A spacer layer 152 is provided between a three-dimensional cell population 8a and a cell culture carrier 150 present in a cell culture section 11. Thus, cells are difficult to be affected by heating when an external signal is input.

According to the cell culture container in the embodiment, since a portion other than the culture space is small, it is possible to secure a wide culture space and to culture a large amount of cells. Furthermore, since the cell culture container does not have a complicated structure, it is possible to produce the cell culture container at low cost. In addition to this, since an input of an external signal is adjusted, it is possible to control an amount of active substances, a timing at which an active substance is added, a type of active substance, and the like in detail.

The cell culture container in the embodiment is appropriate for cell production of a regenerative medicine product which requires highly efficient mass production by culturing a complicated protocol for a long period of time.

For example, in the differentiation of iPS cells into hepatocytes, it is necessary to perform long-term culture for 30 days or longer while changing a type of cytokine of about 3 to 5 types in accordance with the day. Furthermore, in the case of assuming transplantation treatment for liver failure, the required number of cells is calculated to be about 10⁹ to 10¹⁰ and high-density and efficient cell production is desired. The cell culture container in the embodiment is not a constitution in which wiring and a flow path are essential and can also cope with a complicated protocol requiring various active substances by a simple mechanism and a large amount of culture is possible.

### [Examples]

The present invention will be described in more detail below using examples.

### □Examples 1□

Materials used in Examples 1 are as follows.

### • Porous substrate

As a porous substrate, 4% agarose gel particles (CarboxyLink: Thermo Fisher Scientific) whose surface had a carboxyl group were used.

### • Stimulus-responsive polymer

A stimulus-responsive polymer, poly(allylamine-co-allylurea) gel were used. The gel was prepared by introducing an ureido group into a primary amine of 93% allylamine with respect to poly(allylamine) having a molecular weight of 15,000 (Nittobo Medical) with reference to pages 182 to 184 of DDS carrier preparation protocol collection (CMC Publishing). Since the ureido group also contains an amino group, glutaraldehyde was able to form a gel by cross-linking only some intra- and intermolecular amino groups.

The gel had a rich amino group. For this reason, the partners of these hydrogen bonds could be switched between an amino group in a poly(allylamine-co-allylurea) molecule and a water molecule in a solvent in accordance with a temperature and a phase transition of aggregation and swelling could occur.

The phase transition temperature of the gel was 44 °C under physiological conditions such as a NaCl concentration of 150 mM and pH of 7.5. Swelling occurred when a temperature is equal to or higher than the phase transition temperature, the permeability of a substance is increased, and the solubility of the gel in water is increased.

### • External signal receiving material

As an external signal receiving material, gold nanorods (Sigma-Aldrich) which had an absorption peak at a wavelength of 808 nm, whose surfaces were modified with an amino group, and which had a diameter of 10 nm were used.

### • External signal

A high power fiber output LD light source of 808 nm (As One) was used as an external signal source.

### • Cell accommodation section

A TC-treated 96-well plate (Corning) was used as a cell accommodation section.

### • Active substance and cultured cells

VEGF-A which was a vascular endothelial cell growth factor was used as an active substance.

Human umbilical vein endothelial cells HUVEC were used as cultured cells. HUVEC was used for physiological and pharmacological tests such as blood coagulation and angiogenesis. An EBM™ endothelial cell basal medium (LONZA) to which VEGF was not added was used as a medium.

### • Production of cell culture carrier

A carboxyl group on surfaces of agarose gel particles of a porous substrate were activated with EDC and sulfo-NHS. An activation reaction was performed at room temperature in an MES buffer of pH 6.0. Thus, a substance containing an amino group can be immobilized above the agarose gel particles due to a covalent bond.

After the obtained agarose gel particles were washed with an MES buffer, amino group-modified gold nanorods were caused to react in a phosphate buffered saline of pH 7.2.

Subsequently, the synthesized poly(allylamino-co-allylurea) gel was reacted to immobilize the gold nanorods and poly(allylamine-co-allylurea) gel on the surface of the agarose gel particles through some amino groups in the gel. Unreacted gold nanorods and gel were removed through washing. Thus, Composite 1 of a stimulus-responsive substrate for cell culture and a porous substrate was formed.

The obtained Composite 1 was immersed in a 10 mM HEPES buffer of pH 7.5 to which a 10 mM NaCl was added.

Under the conditions, a phase transition temperature was reduced, a stimulus-responsive polymer was swelled at room temperature, and it is possible to take an active substance.

A supernatant was removed so that particles remained and a HEPES buffer in which VEGF was dissolved was added as an active substance. VEGF passed through a stimulus-responsive polymer in a swelled state through diffusion and was incorporated into the porous substrate. Thus, a cell culture carrier was produced.

Subsequently, a cell culture carrier was transferred to a medium. Thus, since a NaCl concentration was increased and a phase transition temperature reached 40 °C or higher, VEGF could be retained in a porous substrate. VEGF-incorporated particles were stored at 4 °C until it was used.

### • Cell culture

HUVEC suspended in a medium was seeded on a culture plate and cells were adhered by culturing it at 37 °C and 5% CO₂ in an incubator.

After the medium was replaced with a new medium, a cell culture carrier was introduced. A stimulus-responsive polymer was locally heated through irradiation of near-infrared light and was swelled and the solubility of the polymer in water is increased.

Along with this, if the contained VEGF was released into a medium through diffusion, HUVEC accelerated the proliferation. After an amount of diffusion of VEGF reached a required amount, if the irradiation of near-infrared light was stopped to prevent VEGF from affecting the experimental results by acting on cell metabolism, the release of VEGF could be stopped.

### [Reference Signs List]

- 20, 30, 40, 50, 70, 80, 92, 100, 110, 132, 140: Cell culture carrier
- 21, 33, 51, 61, 90, 103: Stimulus-responsive substrate for cell culture
- 22, 32, 52, 62, 91, 101: Active substance
- 31: Active substance impermeable layer
- 34, 62, 101: Porous substrate
- 62: Porous substrate containing active substance
- 11: Cell culture section
- 1, 60, 71, 133: Cell culture container
- 2, 14: Cell accommodation section
- 3, 5: Cell culture carrier
- 6: Pipette
- 6a: Suspension solution
- 7: Lid
- 8: Cell population
- 8a: Three-dimensional cell population
- 9: Cell inducing factor
- 10: Growth factor
- 131: Semi-permeable film
- 130: Medium

## Claims

1. A cell culture carrier comprising:
a stimulus-responsive substrate for cell culture comprising a stimulus-responsive polymer and an external signal receiving material, an active substance is supported on the stimulus-responsive substrate for cell culture.

2. The cell culture carrier according to claim 1,
wherein the active substance is contained in a porous substrate, and the porous substrate and the active substance are supported in the stimulus-responsive substrate for cell culture.

3. The cell culture carrier according to claim 1 or 2,
wherein the stimulus-responsive polymer is a temperature responsive polymer.

4. The cell culture carrier according to any one of claims 1 to 3,
wherein the external signal receiving material is an organic compound, a metal structure, or a carbon material.

5. The cell culture carrier according to claim 4,
wherein the metal structure is gold nanorods.

6. The cell culture carrier according to any one of claims 1 to 5,
wherein the active substance is a protein such as an enzyme, a cytokine, a differentiation inducing factor, and an antibody, a peptide such as a hormone, a small-molecular compound such as an antibiotic, an amino acid, glucose, and retinoic acid, a nucleic acid such as DNA, nanoparticles, or liposomes.

7. The cell culture carrier according to any one of claims 1 to 6,
wherein an external signal received by the external signal receiving material is near-infrared light.

8. The cell culture carrier according to claim 2,
wherein a part of the porous substrate containing the active substance is covered with the stimulus-responsive substrate for cell culture and the other parts are covered with an active substance impermeable layer.

9. The cell culture carrier according to claim 1,
wherein the external signal receiving material is blended with the stimulus-responsive substrate for cell culture so that a concentration distribution is formed.

10. The cell culture carrier according to claim 1, further comprising:
an injection port through which the active substance is injected; and
a sealing section formed of a material impermeable to an active substance and configured to seal the injection port are provided.

11. A cell culture container comprising:
a cell culture carrier comprising a stimulus-responsive substrate for cell culture comprising a stimulus-responsive polymer and an external signal receiving material, an active substance is supported on the stimulus-responsive substrate for cell culture; and
a cell culture section.

12. The cell culture container according to claim 11,
wherein the active substance is contained in a porous substrate and the porous substrate and the active substance are supported in the stimulus-responsive substrate for cell culture.

13. The cell culture container according to claim 11 or 12,
wherein the stimulus-responsive polymer is a temperature responsive polymer.

14. The cell culture container according to any one of claims 11 to 13,
wherein the external signal receiving material is an organic compound, a metal structure, or a carbon material.

15. The cell culture container according to claim 14,
wherein the metal structure is gold nanorods.

16. The cell culture container according to any one of claims 11 to 15,
wherein the active substance is a protein such as an enzyme, a cytokine, a differentiation inducing factor, and an antibody, a peptide such as a hormone, a small-molecular compound such as an antibiotic, an amino acid, glucose, and retinoic acid, a nucleic acid such as DNA, nanoparticles, or liposomes.

17. The cell culture container according to any one of claims 11 to 16,
wherein an external signal received by the external signal receiving material is near-infrared light.

18. The cell culture container according to claim 12,
wherein a part of the porous substrate containing the active substance is covered with the stimulus-responsive substrate for cell culture and the other parts are covered with an active substance impermeable layer.

19. The cell culture container according to claim 11,
wherein the external signal receiving material is blended with the stimulus-responsive substrate for cell culture so that a concentration distribution is formed.

20. The cell culture container according to claim 11, further comprising:
an injection port through which the active substance is injected; and
a sealing section formed of a material impermeable to an active substance and configured to seal the injection port are provided.
